(19) **Europäisches Patentamt European Patent Office Office européen des brevets**

(11) **EP 1 346 215 B1**

(12) **FASCICULE DE BREVET EUROPEEN**

(45) Date de publication et mention de la délivrance du brevet:
**31.08.2011 Bulletin 2011/35**

(21) Numéro de dépôt: **01990600.7**

(22) Date de dépôt: **20.12.2001**

(51) Int Cl.:
***G01N 33/22*** *(2006.01)*

(86) Numéro de dépôt international:
**PCT/FR2001/004125**

(87) Numéro de publication internationale:
**WO 2002/052258 (04.07.2002 Gazette 2002/27)**

(54) **PROCEDE ET DISPOSITIF D'EVALUATION DE L'INDICE DE WOBBE D'UN GAZ COMBUSTIBLE**

VERFAHREN UND EINRICHTUNG ZUR BEWERTUNG EINES BRENNSTOFFGAS-WOBBE-INDEX

METHOD AND DEVICE FOR EVALUATING A FUEL GAS WOBBE INDEX

(84) Etats contractants désignés:
**AT BE CH CY DE DK ES FI FR GB GR IE IT LI LU MC NL PT SE TR**

(30) Priorité: **26.12.2000 FR 0017048**

(43) Date de publication de la demande:
**24.09.2003 Bulletin 2003/39**

(73) Titulaire: **GDF SUEZ**
**92400 Courbevoie (FR)**

(72) Inventeurs:
• **CORDIER, Rémy**
**F-92330 Sceaux (FR)**
• **LANTOINE, Laurent**
**F-95220 Herblay (FR)**

(74) Mandataire: **Thinat, Michel**
**Cabinet Weinstein**
**56 A, rue du Faubourg Saint-Honoré**
**75008 Paris (FR)**

(56) Documents cités:
**EP-A- 0 469 649    DE-A- 4 118 781**
**FR-A- 2 792 415    US-A- 4 384 792**

**Description**

L'invention concerne, de façon générale, le domaine des techniques de mesure de l'indice de Wobbe, cet indice étant représenté par le rapport du pouvoir calorifique d'un gaz combustible sur la racine carrée de la densité de ce gaz.

Plus précisément, l'invention, selon un premier de ses aspects, concerne un procédé d'évaluation de l'indice de Wobbe d'un gaz combustible appartenant à une famille de gaz définie par des constituants de nature chimique déterminée intervenant dans des proportions relatives variables, ce procédé comprenant une procédure de mesure au cours de laquelle est effectuée une mesure de débit de ce gaz combustible.

L'indice de Wobbe constitue la principale grandeur caractéristique d'un combustible gazeux et intervient à ce titre sur les réglages de combustion des brûleurs à gaz.

Ainsi, toutes choses étant égales par ailleurs, le débit calorifique d'un brûleur est proportionnel à l'indice de Wobbe, et son excès d'air en dépend directement.

Les réseaux de transport et de distribution de gaz naturel étant de plus en plus maillés et alimentés par des sources d'énergie diverses, l'indice de Wobbe d'un gaz de type donné peut varier dans des proportions non négligeables, par exemple de +/- 5% en un point donné d'un réseau.

Or, certains procédés industriels, dans les industries du verre et de la chaux notamment, sont sensibles à ces variations au point de nécessiter la mise en oeuvre de solutions de régulation de combustion spécifiques, l'une de ces solutions consistant à intégrer le résultat d'une mesure locale de l'indice de Wobbe dans les algorithmes de régulation.

A ce jour, tous les appareils de mesure de l'indice de Wobbe disponibles sur le marché sont relativement complexes, et par conséquent d'un coût élevé.

Trois principes sont connus pour déterminer l'indice de Wobbe.

Le premier principe consiste à associer une mesure de pouvoir calorifique, obtenue par calorimétrie ou chromatographie, à une mesure de densité, obtenue par densitométrie ou par chromatographie.

Le second principe consiste à analyser les produits de combustion du gaz concerné dans un petit four où un échantillon de ce gaz est brûlé à la stoechiométrie ou en excès d'air.

Le troisième principe consiste à mesurer des caractéristiques physiques du gaz, telles que viscosité, capacité calorifique, etc..., et à faire une corrélation entre ces mesures et l'indice de Wobbe.

Deux exemples de mise en oeuvre de ce troisième principe sont décrits dans les documents de brevets DE - 41 18 781 et US - 4 384 792.

Le document DE - 41 18 781 décrit en fait un procédé visant à d'autres mesures en plus de celle de l'indice de Wobbe, ayant recours à deux fonctions de corrélation à quatre constantes, utilisant trois mesures de débit, une mesure de pression différentielle et une mesure de température, et requérant un étalonnage au méthane.

Le document US - 4 384 792 décrit un procédé de mesure de l'indice de Wobbe, ayant recours à une fonction de corrélation à trois constantes, utilisant une mesure de débit volumique, une mesure de pression différentielle et une mesure de température, et requérant un régulateur de pression et un étalonnage au moyen d'un gaz hydrogéné.

Par ailleurs, un dispositif pour l'évaluation de l'indice de Wobbe est connu du document US-A-6 119 710.

Ces techniques connues présentent donc une relative complexité, le but de l'invention est de proposer un procédé de mesure de l'indice de Wobbe plus aisé à mettre en oeuvre et, corrélativement, un dispositif sensiblement moins complexe et moins coûteux que les dispositifs connus.

A cette fin, le procédé de l'invention, par ailleurs conforme à la définition générique qu'en donne le préambule ci-dessus, est essentiellement caractérisé en ce qu'il comprend : une opération fournissant, en tant que mesure de débit du gaz combustible, une mesure d'un débit massique de ce gaz combustible en écoulement sonique à travers une restriction fluidique, telle qu'un orifice ou une micro-tuyère, effectuée à une pression absolue de mesure et à une température absolue de mesure; une procédure d'étalonnage au cours de laquelle est effectuée une mesure d'un débit massique d'un gaz de référence en écoulement sonique à travers la restriction fluidique, à une pression absolue de référence et à une température absolue de référence; et une procédure d'évaluation au cours de laquelle l'indice de Wobbe reçoit une valeur liée, par une loi affine empirique préalablement établie pour la famille de gaz, au produit de trois facteurs, le premier facteur représentant le rapport de la mesure du débit massique du gaz combustible à la mesure du débit massique du gaz de référence, le second facteur représentant le rapport d'images respectives des pressions absolues de référence et de mesure par une fonction polynomiale déterminée, et le troisième facteur représentant la racine carrée du rapport des températures absolues de mesure et de référence.

Dans le cas où le gaz combustible et le gaz de référence peuvent avoir des pressions différentes, la procédure de mesure comprend une mesure de la pression absolue de mesure, et la procédure d'étalonnage comprend une mesure de la pression absolue de référence.

Si les pressions absolues de mesure et de référence sont maintenues dans une plage prédéterminée, la fonction polynomiale peut être assimilée à la fonction identité, l'image d'une pression (P) par la fonction polynomiale déterminée prenant ainsi la forme :

$$F(P) = P.$$

Dans le cas contraire, l'image d'une pression (P) par la fonction polynomiale déterminée prend de préférence la forme :

$$F(P) = P - k \cdot P^{(1-r)},$$

où k et r sont des paramètres de construction de la restriction fluidique.

Dans le cas où le gaz combustible et le gaz de référence peuvent avoir des températures différentes, la procédure de mesure comprend une mesure de la température absolue de mesure, et la procédure d'étalonnage comprend une mesure de la température absolue de référence.

Dans ces conditions, le gaz de référence peut être librement choisi parmi un ensemble de gaz comprenant des gaz et mélanges gazeux non combustibles, tels que l'air ou l'azote.

Le procédé de l'invention comprend avantageusement une procédure préliminaire de corrélation, au cours de laquelle la loi affine empirique est établie pour au moins deux gaz de la famille de gaz.

Pour l'évaluation de l'indice de Wobbe d'un gaz de la famille des gaz naturels, la loi affine empirique est définie par une ordonnée à l'origine égale à -18,40.

Dans ce cas, et si l'air comprimé est utilisé comme gaz de référence, la loi affine empirique est définie par une pente égale à 19,40.

Si en revanche l'azote est utilisé comme gaz de référence, la loi affine empirique est définie par une pente égale à 19,72.

Si, toujours pour l'évaluation de l'indice de Wobbe d'un gaz de la famille des gaz naturels, le méthane est utilisé comme gaz de référence, la loi affine empirique est définie par une pente égale à 33,28.

L'invention concerne également un dispositif pour la mise en oeuvre du procédé d'évaluation de l'indice de Wobbe tel que précédemment décrit, ce dispositif étant caractérisé en ce qu'il comprend une conduite présentant une entrée et une sortie, des moyens d'admission pour guider sélectivement, jusqu'à l'entrée de la conduite, un flux de gaz combustible sous pression ou un flux de gaz de référence sous pression, une restriction fluidique, telle qu'un orifice ou une micro-tuyère, présentant une entrée reliée à la sortie de la conduite, et une sortie, et un débitmètre massique relié à la sortie de la restriction fluidique et fournissant un signal de sortie représentatif du débit massique du gaz traversant cette restriction fluidique en écoulement sonique, ainsi qu'une unité logique pour exploiter les signaux de sortie du débit massique du gaz combustible et du débit massique du gaz de référence.

Dans les cas où le gaz de référence et le gaz combustible peuvent être utilisés dans des conditions différentes de pression et / ou de température, le dispositif de l'invention comprend un capteur de pression absolue et / ou un capteur de température absolue installés sur la conduite.

Les moyens d'admission précédemment évoqués comprennent par exemple une première entrée principale pour le gaz combustible, une seconde entrée principale pour le gaz de référence, et des première et seconde électrovalves respectivement interposées entre l'entrée de la conduite, et les première et seconde entrées principales.

Dans ce cas, le dispositif de l'invention est avantageusement équipé d'une unité logique comprenant au moins trois entrées d'acquisition respectivement reliées au débitmètre, au capteur de pression et au capteur de température, et deux sorties de commande respectivement reliées aux électrovalves.

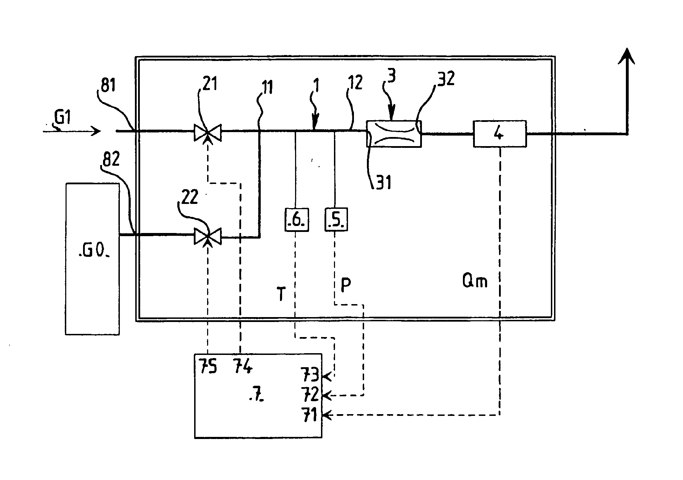

D'autres caractéristiques et avantages de l'invention ressortiront clairement de la description qui en est faite ci-après, à titre indicatif et nullement limitatif, en référence à l'unique figure annexée, constituée par le schéma d'un dispositif conforme à l'invention.

Pour rendre plus concret l'exposé de l'invention, la présente description se référera tout d'abord à la figure annexée, et au dispositif qu'elle représente.

Ce dispositif comprend une conduite 1 dont l'entrée 11 est reliée à une première entrée principale 81 du dispositif par une première électrovalve 21, et reliée à une seconde entrée principale 82 du dispositif par une seconde électrovalve 22.

La première entrée principale 81 du dispositif est reliée en permanence à une source pressurisée du gaz combustible à analyser G1.

La seconde entrée principale 82 du dispositif est reliée en permanence à une source pressurisée d'un gaz de référence G0, tel que de l'air, de l'azote ou du méthane, entre autres possibilités.

Les moyens d'admission que constituent les entrées principales 81 et 82, en combinaison avec les électrovalves 21 et 22, dont chacune est commandée pour être passante pendant que l'autre est fermée, permettent donc d'acheminer à volonté, jusqu'à l'entrée 11 de la conduite 1, un flux du gaz combustible G1 sous pression, ou un flux du gaz de référence G0 sous pression.

Le dispositif comprend par ailleurs une restriction fluidique 3 dont l'entrée 31 est reliée à la sortie 12 de la conduite 1, et un débitmètre massique 4 relié à la sortie 32 de la restriction fluidique 3.

La restriction fluidique 3, destinée à être le siège d'un écoulement sonique du gaz G0 ou G1 qui la traverse, et à offrir une résistance à cet écoulement, prend typiquement la forme d'un orifice ou une micro-tuyère dont le diamètre est par exemple de l'ordre de 0,4 millimètres.

Le débitmètre massique 4, connu en soi et par exemple dimensionné pour environ 300 litres normaux par heure, fournit un signal de sortie Qm représentatif du débit massique du gaz G0 ou G1 qui traverse la restriction fluidique 3 en écoulement sonique.

Dans le mode de réalisation permettant la plus grande précision de mesure, le dispositif de l'invention comprend également un capteur 5 de pression absolue et un capteur 6 de température absolue, tous deux installés sur la conduite 1, c'est-à-dire disposés de manière à fournir des signaux de mesure respectifs P et T, respectivement représentatifs de la pression et de la température régnant dans cette conduite, la pression P étant typiquement inférieure à 5 bar.

La commande des électrovalves 21 et 22, et l'exploitation des signaux Qm, P et T, peuvent être confiées à une unité logique 7, qui comprend trois entrées d'acquisition 71, 72, et 73, respectivement reliées au débitmètre 4, au capteur de pression 5 et au capteur de température 6, et deux sorties de commande 74 et 75, respectivement reliées aux électrovalves 21 et 22.

Le procédé de l'invention, qui est mis en oeuvre dans ce dispositif, permet d'évaluer l'indice de Wobbe de n'importe quel gaz combustible tel que G1, sous réserve que soit identifiée la famille de gaz à laquelle appartient ce gaz, telle qu'elle est définie par les principaux constituants chimiques de ce gaz, même s'ils interviennent dans des proportions variables, et sous réserve de disposer, sur cette famille de gaz, de connaissances préalables qui seront précisées ultérieurement.

Ce procédé comprend les opérations suivantes.

Tout d'abord, l'un quelconque des gaz à utiliser, par exemple le gaz combustible G1, est autorisé à traverser la restriction 3 en écoulement sonique, et le signal Qm1 alors délivré par le débitmètre 4 est pris en compte comme mesure de débit massique du gaz combustible G1 dans la restriction 3.

Corrélativement, les signaux P1 et T1 respectivement délivrés pendant le même temps par les capteurs 5 et 6 sont pris en compte comme mesures respectives de pression absolue de mesure et de température absolue de mesure.

Le second des gaz à utiliser, en l'occurrence le gaz de référence G0, est ensuite autorisé à traverser la restriction 3 en écoulement sonique, et le signal Qm0 alors délivré par le débitmètre 4 est pris en compte comme mesure de débit massique du gaz de référence G0 dans la restriction 3.

Corrélativement, les signaux P0 et T0 respectivement délivrés pendant le même temps par les capteurs 5 et 6 sont pris en compte comme mesures respectives de pression absolue de référence et de température absolue de référence.

Cette phase de mesure est suivie d'une phase d'évaluation au cours de laquelle sont évalués au moins trois facteurs, qui seront notés Za, Zb, et Zc.

Le premier facteur Za est représenté par le rapport Qm1/Qm0 des signaux de sortie du débitmètre 4 pour les gaz G1 et G0, c'est-à-dire par le rapport de la mesure du débit massique Qm1 du gaz combustible G1 à la mesure du débit massique Qm0 du gaz de référence G0.

Le second facteur Zb est représenté par le rapport F(P0) / F(P1) de l'image F(P0) de la pression absolue de référence P0 par une fonction polynomiale déterminée F, qui sera précisée ultérieurement, à l'image F(P1) de la pression absolue de mesure P1 par la fonction polynomiale F.

En pratique, si les pressions P0 et P1 sont proches l'une de l'autre, et par exemple si elles ne présentent entre elles qu'une différence relative maximum de l'ordre de 2% à 3%, la fonction polynomiale F est assimilable à la fonction identité, c'est-à-dire que le rapport F(P0) / F(P1) est simplement assimilable au rapport P0 / P1.

Et le troisième facteur Zc est représenté par la racine carrée $(T1/T0)^{1/2}$ du rapport T1/T0 de la température absolue de mesure T1 à la température absolue de référence T0.

Dans ces conditions, le procédé de l'invention attribue au gaz combustible G1, comme indice de Wobbe, la valeur W définie par une loi affine empirique de la forme :

$$W = A \times Y + B,$$

dans laquelle le terme Y représente le produit Za x Zb x Zc des trois facteurs Za, Zb, et Zc, et dans laquelle le coefficient A, appelé "pente", et le coefficient B, appelé. "ordonnée à l'origine", sont préalablement établis, pour les gaz de la famille de gaz considérée, d'une façon qui sera précisée ultérieurement.

Si le dispositif comporte des moyens permettant de rendre P1 égal à P0 et T1 égal à T0, c'est-à-dire si :

$$(P0 \; / \; P1) \; = \; 1 \quad et \quad (T1/T0)^{\frac{1}{2}} \; = \; 1,$$

le facteur Za pourra directement être assimilé au terme Y, comme le montrent aisément les relations ci-dessus et le fait que les facteurs Zb et Zc sont alors tous deux égaux à 1.

Dans le cas où les pressions P0 et P1 sont en revanche différentes l'une de l'autre, et présentent par exemple entre elles une différence relative supérieure à 3%, la fonction polynomiale F n'est plus assimilable à la fonction identité, c'est-à-dire que l'image F(P) d'une pression P par cette fonction F n'est plus assimilable à la pression P elle-même, mais doit être corrigée d'un facteur noté $C_D$ et égal à 1 - k . P$^{-r}$.

Le facteur noté $C_D$ permet en fait de tenir compte de l'effet qu'a la couche limite, due à la viscosité du gaz qui traverse la restriction 3, sur les caractéristiques d'écoulement de ce gaz, les paramètres k et r intervenant dans le facteur $C_D$ étant des paramètres de construction de la restriction fluidique 3 qui peuvent soit être fournis par le fabricant de cette restriction, soit être déterminés par des techniques bien connues de l'homme de métier.

Dans un exemple particulier de réalisation de l'invention, le facteur $C_D$ pour une restriction déterminée a pu être assimilé à 1 - 0,881. P$^{-4,9}$.

Quelles que soient les valeurs particulières de k et r, l'image d'une pression P par la fonction polynomiale F prend donc la forme :

$$F(P) \; = \; P \; - \; k \; . \; P^{1-r},$$

et le second facteur Zb, qui est représenté par le rapport F(P0) / F(P1), prend la forme :

$$Zb \; = \; (P0 \; - \; k \; . \; P0^{1-r}) \; / \; (P1 \; - \; k \; . \; P1^{1-r}).$$

Dans le cas de l'évaluation de l'indice de Wobbe d'un gaz de la famille des gaz naturels, l'ordonnée à l'origine B de la loi affine empirique W = A x Y + B prend la valeur - 18,40.

Si par ailleurs, pour cette même application, l'air comprimé est utilisé comme gaz de référence G0, la pente A de la loi affine empirique W = A x Y + B prend la valeur 19,40, cette loi étant donc globalement définie par la relation :

$$W \; = \; 19,40 \; X \; Y \; - \; 18,40,$$

c'est-à-dire encore par :

$$W \; = \; 19,40 \; X \; Za \; X \; Zb \; X \; Zc \; - \; 18,40.$$

Dans le cas de l'évaluation de l'indice de Wobbe d'un gaz de la famille des gaz naturels, et de l'utilisation d'azote comme gaz de référence G0, la pente A de la loi affine empirique W = A x Y + B prend la valeur . 19,72, cette loi étant donc globalement définie par la relation :

$$W \; = \; 19,72 \; X \; Y \; - \; 18,40,$$

c'est-à-dire encore par :

$$W \; = \; 19,72 \; X \; Za \; X \; Zb \; X \; Zc \; - \; 18,40.$$

Dans le cas de l'évaluation de l'indice de Wobbe d'un gaz de la famille des gaz naturels, et de l'utilisation de méthane pur comme gaz de référence G0, la pente A de la loi affine empirique W = A x Y + B prend la valeur 33,28, cette loi étant donc globalement définie par la relation :

$$W = 33,28 \times Y - 18,40,$$

c'est-à-dire encore par :

$$W = 33,28 \times Za \times Zb \times Zc - 18,40.$$

Comme le montrent les exemples précédents, l'invention permet le libre choix du gaz de référence G0, de sorte qu'il est possible d'utiliser, comme gaz de référence, des gaz et mélanges gazeux non combustibles et donc peu coûteux, tels que l'air ou l'azote.

Dans le cas de l'évaluation de l'indice de Wobbe d'un gaz n'appartenant pas à la famille des gaz naturels, et / ou de l'utilisation, en tant que gaz de référence G0, d'un gaz différent de ceux pour lesquels la loi affine empirique W = A x Y + B a été définie ci-dessus, le procédé de l'invention doit inclure une procédure préliminaire de corrélation, au cours de laquelle la loi affine empirique W = A x Y + B est établie pour au moins deux gaz G11 de la nouvelle de famille de gaz combustibles considérée, et / ou pour au moins un gaz de référence G0 tel que l'air, l'azote ou le méthane, ou pour un autre gaz de référence G00.

Cette procédure préliminaire de corrélation peut aisément être mise en oeuvre d'une part en mesurant les facteurs tels que Zai, Zbi, et Zci, respectivement obtenus pour différents gaz combustibles G1i de la nouvelle famille de gaz avec un même gaz de référence, d'autre part en mesurant directement, par une technique différente de celle de l'invention et par exemple par l'une des techniques traditionnelles connues, les indices de Wobbe tels que Wi de ces mêmes gaz combustibles, et enfin en résolvant, de façon bien connue en soi, le système d'équations du premier degré du type Wi = A x Zai x Zbi x Zci + B pour obtenir les valeurs à donner aux paramètres A et B, a priori inconnus, qui doivent être utilisés conformément au procédé de l'invention pour cette nouvelle famille de gaz.

D'un point de vue physique, la mesure de débit massique Qm pour un gaz tel que G0 ou G1, réalisée dans les conditions exposées précédemment en référence à la figure, est liée à une grandeur dite "débit massique normal" et notée Q par la relation :

$$Qm = Q / C,$$

dans laquelle C est un coefficient correcteur qui dépend de diverses propriété physiques du gaz réel pour lequel la mesure est effectuée, et plus précisément de sa capacité calorifique, de sa viscosité, et de sa conductivité thermique.

Pour un gaz de composition connue, le coefficient correcteur C de ce gaz est lié aux différents coefficients correcteurs Cj de ses constituants par la relation :

$$(1 / C) = \Sigma (Xj / Cj),$$

les différents coefficients Cj étant donnés dans des tables établies par les fabricants de débitmètres massiques, et chaque Xj représentant la fraction volumique du constituant j.

Le débit massique normal Q satisfait lui-même la relation :

$$Q = k \times C_D \times C_R \times P / (T \times d)^{\frac{1}{2}}$$

dans laquelle :

k est un paramètre de construction de la restriction 3, déjà évoqué,

$C_D$ est un facteur de correction de la forme $1 - k \cdot P^{-r}$, déjà évoqué,

P est la pression absolue du gaz considérée, telle qu'elle est mesurée par le capteur 5,

T est la température absolue du gaz considérée, telle qu'elle est mesurée par le capteur 6,

d est la densité du gaz considéré, et

$C_R$ est le coefficient de gaz réel de la restriction 3, qui ne dépend, pour le gaz réel qui fait l'objet des mesures, que du rapport $\gamma$ de la chaleur spécifique $C_P$ de ce gaz à pression constante, à sa chaleur spécifique $C_V$ à volume constant, le coefficient $C_R$ prenant la forme :

$$C_R = (\gamma)^{\frac{1}{2}} \times (2 / (\gamma + 1))^{((\gamma + 1) / 2 \times (\gamma - 1))},$$

le rapport $\gamma$ étant typiquement de l'ordre de 0, 67 pour le méthane et les gaz naturels, et de 0,69 pour l'air et l'azote.

Dans ces conditions, les relations ci-dessus permettent de montrer que :

$$Y = (Qm1 / Qm0) \times (C_{D0} / C_{D1}) \times (P0 / P1) \times (T1/T0)^{\frac{1}{2}},$$

et que :

$$Y = (C0 / C1) \times (C_{R1} / C_{R0}) \times (d0/d1)^{\frac{1}{2}},$$

où C0 et C1 désignent respectivement le coefficient correcteur C pour le gaz de référence G0 et pour le gaz combustible G1, où $C_{R1}$ et $C_{R0}$ désignent respectivement le coefficient de gaz réel de la restriction 3 pour le gaz combustible G1 et pour le gaz de référence G0, et où d0 et d1 désignent respectivement la densité du gaz de référence G0 et celle du gaz combustible G1.

D'un point de vue physique, le procédé de l'invention peut donc être analysé comme reposant sur la mise en évidence d'une loi affine empirique liant l'indice de Wobbe W de chaque gaz combustible à chacune des expressions ci-dessus du terme Y.

**Revendications**

1. Procédé d'évaluation de l'indice de Wobbe d'un gaz combustible (G1) appartenant à une famille de gaz définie par des constituants de nature chimique déterminée intervenant dans des proportions relatives variables, ce procédé comprenant une procédure de mesure au cours de laquelle est effectuée une mesure de débit de ce gaz combustible, **caractérisé en ce qu'**il comprend :

   une opération fournissant, en tant que mesure de débit du gaz combustible, une mesure (Qm1) d'un débit massique de ce gaz combustible (G1) en écoulement sonique à travers une restriction fluidique (3), telle qu'un orifice ou une micro-tuyère, effectuée à une pression absolue de mesure (P1) et à une température absolue de mesure (T1),
   une procédure d'étalonnage au cours de laquelle est effectuée une mesure (Qm0) d'un débit massique d'un gaz de référence (G0) en écoulement sonique à travers la restriction fluidique (3), à une pression absolue de référence (P0) et à une température absolue de référence (T0), et
   une procédure d'évaluation au cours de laquelle l'indice de Wobbe reçoit une valeur (W) liée, par une loi affine empirique (W = A x Y + B) préalablement établie pour la famille de gaz, au produit (Y) de trois facteurs (Za, Zb, Zc), le premier facteur (Za) représentant le rapport (Qm1/Qm0) de la mesure du débit massique (Qm1) du gaz combustible (G1) à la mesure du débit massique (Qm0) du gaz de référence (G0), le second facteur (Zb) représentant le rapport (F(P0)/F(P1)) d'images respectives (F(P0), F(P1)) des pressions absolues de référence (P0) et de mesure (P1) par une fonction polynomiale déterminée (F), et le troisième facteur (Zc) représentant la racine carrée (($T1/T0)^{1/2}$) du rapport (T1/T0) des températures absolues de mesure (T1) et de référence (T0) .

2. Procédé d'évaluation de l'indice de Wobbe suivant la revendication 1, **caractérisé en ce que** la procédure de mesure comprend une mesure de la pression absolue de mesure (P1), et **en ce que** la procédure d'étalonnage comprend une mesure de la pression absolue de référence (P0).

3. Procédé d'évaluation de l'indice de Wobbe suivant la revendication 2, **caractérisé en ce que** les pressions absolues de mesure et de référence (P1, P0) sont maintenues dans une plage prédéterminée, et **en ce que** la fonction polynomiale (F) est constituée par la fonction identité, l'image d'une pression (P) par la fonction polynomiale déterminée (F) prenant ainsi la forme :

$$F(P) = P .$$

**4.** Procédé d'évaluation de l'indice de Wobbe suivant la revendication 2, **caractérisé en ce que** l'image d'une pression (P) par la fonction polynomiale déterminée (F) prend la forme :

$$F(P) = P - k \cdot P^{(1-r)},$$

où k et r sont des paramètres de construction de la restriction fluidique (3).

**5.** Procédé d'évaluation de l'indice de Wobbe suivant l'une quelconque des revendications précédentes, **caractérisé en ce que** la procédure de mesure comprend une mesure de la température absolue de mesure (T1), et **en ce que** la procédure d'étalonnage comprend une mesure de la température absolue de référence (T0).

**6.** Procédé d'évaluation de l'indice de Wobbe suivant l'une quelconque des revendications précédentes, **caractérisé en ce que** le gaz de référence (G0) est librement choisi parmi un ensemble de gaz comprenant des gaz et mélanges gazeux non combustibles, tels que l'air ou l'azote.

**7.** Procédé d'évaluation de l'indice de Wobbe suivant l'une quelconque des revendications précédentes, **caractérisé en ce qu'**il comprend une procédure préliminaire de corrélation, au cours de laquelle la loi affine empirique (W = A x Y + B) est établie pour au moins deux gaz de la famille de gaz.

**8.** Procédé d'évaluation de l'indice de Wobbe suivant l'une quelconque des revendications précédentes, **caractérisé en ce que**, pour l'évaluation de l'indice de Wobbe d'un gaz de la famille des gaz naturels, la loi affine empirique (W = A x Y + B) est définie par une ordonnée à l'origine (B) égale à -18,40.

**9.** Procédé d'évaluation de l'indice de Wobbe suivant la revendication 8, **caractérisé en ce que** l'air comprimé est utilisé comme gaz de référence (G0), et **en ce que** la loi affine empirique (W = A x Y + B) est définie par une pente (A) égale à 19,40.

**10.** Procédé d'évaluation de l'indice de Wobbe suivant la revendication 8, **caractérisé en ce que** l'azote est utilisé comme gaz de référence (G0) , et **en ce que** la loi affine empirique (W = A x Y + B) est définie par une pente (A) égale à 19,72.

**11.** Procédé d'évaluation de l'indice de Wobbe suivant la revendication 8, **caractérisé en ce que** le méthane est utilisé comme gaz de référence (G0), et **en ce que** la loi affine empirique (W = A x Y + B) est définie par une pente (A) égale à 33,28.

**12.** Dispositif adapté pour l'évaluation de l'indice de Wobbe d'un gaz combustible (G1) appartenant à une famille de gaz définie par des constituants de nature chimique déterminée intervenant dans des proportions relatives variables, le dispositif comprenant une conduite (1) présentant une entrée (11) et une sortie (12), des moyens d'admission (21, 22, 81, 82) pour guider sélectivement jusqu'à l'entrée (11) de la conduite, un flux de gaz combustible (G1) sous pression ou un flux de gaz de référence (G0) sous pression, une restriction fluidique (3) telle qu'un orifice ou une micro-tuyère, présentant une entrée (31) reliée à la sortie (12) de la conduite (1), et une sortie (32), ainsi qu'un capteur (5) de pression absolue et un capteur (6) de température absolue installés sur la conduite (1) **caractérisé en ce qu'**il comprend un débitmètre massique (4) relié à la sortie (32) de la restriction fluidique (3) et fournissant un signal de sortie (Qm) représentatif du débit massique (4) du gaz traversant cette restriction fluidique (3) en écoulement sonique, ainsi qu'une unité logique (7) pour exploiter les signaux de sortie (Qm1, Qm0, P1, P0, T1, T0) respectivement du flux massique du gaz combustible (G1), du flux massique du gaz de référence (G0) de la pression absolue de mesure, de la pression absolue de référence, de la température absolue de mesure et de la température absolue de référence du gaz combustible et du gaz de référence,

**13.** Dispositif suivant la revendication 12, **caractérisé en ce que** les moyens d'admission (21, 22, 81, 82) comprennent une première entrée principale (81) pour le gaz combustible (G1), une seconde entrée principale (82) pour le gaz de référence (G0), et des première et seconde électrovalves (21, 22) respectivement interposées entre l'entrée (11) de la conduite (1), et les première et seconde entrées principales (81, 82).

**14.** Dispositif suivant la revendication 12 ou 13, **caractérisé en ce que** l'unité logique (7) comprend au moins trois entrées d'acquisition (71, 72, 73) respectivement reliées au débitmètre (4), au capteur de pression (5) et au capteur de température (6), et deux sorties de commande (74, 75) respectivement reliées aux électrovalves (21, 22).

**Claims**

1. A method for evaluating the Wobbe index of a combustible gas (G1) belonging to a family of gases, defined by constituents with a determined chemical nature occurring in variable relative proportions, this method comprising a measurement procedure, during which a measurement of the combustible gas flow rate is carried out, **characterized in that** it comprises:

   an operation providing as a measurement of the combustible gas flow rate, a measurement (Qm1) of a mass flow rate of this combustible gas (G1) in sonic flow through a fluidic restriction (3), such as an orifice or a micronozzle, carried out at an absolute measurement pressure (P1), and at an absolute measurement temperature (T1),
   a calibration procedure, during which a measurement (Qm0) of a mass flow rate of a reference gas (G0) in sonic flow through the fluidic restriction (3) is carried out at an absolute reference pressure (P0), and at an absolute reference temperature (T0), and
   an evaluation procedure, during which the Wobbe index receives a value (W) related, through an empirical affine law (W = A $\times$ Y + B) established beforehand for the family of gases, to the product (Y) of three factors (Za, Zb, Zc), the first factor (Za) representing the ratio (Qm1/Qm0) of the measurement of the mass flow rate (Qm1) of the combustible gas (G1) to the measurement of the mass flow rate (Qm0) of the reference gas (G0), the second factor (Zb) representing the ratio (F(P0)/F(P1)) of respective images (F(P0), F(P1)) of the absolute reference (P0) and measurement (P1) pressures by a determined polynomial function (F), and the third factor (Zc) representing the square root ($(T1/T0)^{1/2}$) of the ratio (T1/T0) of the absolute measurement (T1) and reference (T0) temperatures.

2. The method for evaluating the Wobbe index according to claim 1, **characterized in that** the measurement procedure comprises a measurement of the absolute measurement pressure (P1), and **in that** the calibration procedure comprises a measurement of the absolute reference pressure (P0).

3. The method for evaluating the Wobbe index according to claim 1, **characterized in that** the absolute measurement and reference pressures (P1, P0) are maintained in a predetermined range, and **in that** the polynomial function (F) is formed by the identity function, the image of a pressure (P) by the determined polynomial function (F) thus assuming the form of:

$$F(P) = P.$$

4. The method for evaluating the Wobbe index according to claim 2, **characterized in that** the image of a pressure (P) by the determined polynomial function (F) assumes the form of:

$$F(P) = P - k . P^{(1-r)},$$

wherein f and r are construction parameters of the fluidic restriction (3).

5. The method for evaluating the Wobbe index according to any of the preceding claims, **characterized in that** the measurement procedure comprises a measurement of the absolute measurement temperature (T1), and **in that** the calibration procedure comprises a measurement of the absolute reference temperature (T0).

6. The method for evaluating the Wobbe index according to any of the preceding claims, **characterized in that** the reference gas (G0) is freely selected from a set of gases, comprising non-combustible gases and gas mixtures, such as air or nitrogen.

7. The method for evaluating the Wobbe index according to any of the preceding claims, **characterized in that** it comprises a preliminarily correlation procedure, during which the empirical affine law (W = A $\times$ Y + B) is established for at least two gases of the family of gases.

8. The method for evaluating the Wobbe index according to any of the preceding claims, **characterized in that**, for evaluating the Wobbe index of a gas from the family of natural gases, the empirical affine law (W = A x Y + B) is defined by an ordinate axis intercept (B) equal to -18.40.

9. The method for evaluating the Wobbe index according to claim 8, **characterized in that** compressed air is used as a reference gas (G0), and **in that** the empirical affine law (W = A $\times$ Y + B) is defined by a slope (A) equal to 19.40.

10. The method for evaluating the Wobbe index according to claim 8, **characterized in that** nitrogen is used as a reference gas (G0), and **in that** the empirical affine law (W = A x Y + B) is defined by a slope (A) equal to 19.72.

11. The method for evaluating the Wobbe index according to claim 8, **characterized in that** methane is used as a reference gas (G0), and **in that** the empirical affine law (W = A $\times$ Y + B) is defined by a slope (A) equal to 33.28.

12. A device adapted for evaluating the Wobbe index of a combustible gas (G1) belonging to a family of gases defined by constituents with a determined chemical nature occurring in variable relative proportions, the device comprising a conduit (1) having an inlet (11) and an outlet (12), admission means (21, 22, 81, 82) for selectively guiding as far as the inlet (11) of the conduit, a pressurized combustible gas (G1) flow or a pressurized reference gas (G0) flow, a fluidic restriction (3) such as an orifice or a micronozzle, having an inlet (31) connected to the outlet (12) of the conduit (1) and a outlet (32), as well as an absolute pressure sensor (5) and an absolute temperature sensor (6) installed in the conduit (1), **characterized in that** it comprises a mass flow meter (4) connected to the outlet (32) of the fluidic restriction (3) and providing an output signal (Qm) representative of the mass flow rate (4) of the gas flowing through this fluidic restriction (3) in sonic flow, as well as a logic unit (7) for utilizing the output signals (Qm1, Qm0, P1, P0, T1, T0) of the mass combustible gas flow (G1), of the mass reference gas flow (G0), of the absolute measurement pressure, of the absolute reference pressure, of the absolute measurement temperature and of the absolute reference temperature of the combustible gas and of the reference gas, respectively.

13. The device according to claim 12, **characterized in that** the admission means (21, 22, 81, 82) comprise a first main inlet (81) for the combustible gas (G1), a second main inlet (82) for the reference gas (G0), and first and second solenoid valves (21, 22) respectively interposed between the inlet (11) of the conduit (1) and the first and second main inlets (81, 82).

14. The device according to claim 12 or 13, **characterized in that** the logic unit (7) comprises at least three acquisition inlets (71, 72, 73) respectively connected to the flow meter (4), to the pressure sensor (5) and to the temperature sensor (6), and two control outputs (74, 75) respectively connected to the solenoid valves (21, 22).

**Patentansprüche**

1. Verfahren zur Bewertung des Wobbeindexes eines Brennstoffgases (G1), das einer Gruppe von Gasen angehört, definiert durch Bestandteile einer bestimmten chemischen Art, die in variable relative Proportionen eingreifen, wobei dieses Verfahren ein Verfahren zur Messung umfasst, im Laufe dessen eine Messung des Durchsatzes dieses Brennstoffgases erfolgt, **dadurch gekennzeichnet, dass** es Folgendes umfasst:

   einen Vorgang der, als Messung des Durchsatzes des Brennstoffgases, eine Messung (Qm1) eines Massedurchsatzes dieses Brennstoffgases (G1) bereitstellt, in Strömung mit Schallgeschwindigkeit über eine Durchflussbeschränkung (3),
   wie z.B. eine Öffnung oder eine Mikrodüse, durchgeführt bei einem absoluten Messdruck (P1) und einer absoluten Messtemperatur (T1),
   ein Verfahren der Eichung, im Laufe dessen eine Messung (Qm0) eines Massedurchsatzes eines Referenzgases (G0) in Strömung mit Schallgeschwindigkeit über die Durchflussbeschränkung (3) mit einem absoluten Referenzdruck (P0) und einer absoluten Referenztemperatur (T0) erfolgt, und
   ein Verfahren der Bewertung, im Laufe dessen der Wobbeindex einen Wert (W) annimmt, verbunden durch ein empirisches affines Gesetz (W = A X Y + B), vorher festgesetzt für die Gruppe von Gasen, mit dem Produkt (Y) mit drei Faktoren (Za, Zb, Zc), wobei der erste Faktor (Za) das Verhältnis (Qm1/Qm0) der Messung des Massedurchsatzes (Qm1) des Brennstoffgases (G1) zur Messung des Massedurchsatzes (Qm0) des Referenzgases (G0) darstellt, der zweite Faktor (Zb) das Verhältnis (F(P0)/F(P1)) von entsprechenden Bildern (F(P0), F(P1)) der absoluten Referenz- (P0) und Messdrücke (P1) durch eine bestimmte polynomiale Funktion (F) darstellt und der dritte Faktor (Zc) die Quadratwurzel ($(T1/T0)^{1/2}$) der Beziehung (T1/T0) der absoluten Mess- (T1) und Referenztemperaturen (T0) darstellt.

2. Verfahren zur Bewertung des Wobbeindexes nach Anspruch 1, **dadurch gekennzeichnet, dass** das Verfahren der Messung eine Messung des absoluten Messdrucks (P1) umfasst, und **dadurch**, dass das Verfahren der Eichung eine Messung des absoluten Referenzdrucks (P0) umfasst.

3. Verfahren zur Bewertung des Wobbeindexes nach Anspruch 2, **dadurch gekennzeichnet, dass** die absoluten Mess- und Referenzdrücke (P1, P0) in einem vorbestimmten Bereich gehalten werden, und **dadurch**, dass die polynomiale Funktion (F) durch die Identitätsfunktion gebildet ist, wobei das Bild eines Drucks (P) durch die bestimmte polynomiale Funktion (F) so die Folgende Form annimmt:

$$F(P) = P .$$

4. Verfahren zur Bewertung des Wobbeindexes nach Anspruch 2, **dadurch gekennzeichnet, dass** das Bild eines Drucks (P) durch die bestimmte polynomiale Funktion (F) die Folgende Form annimmt:

$$F(P) = P - k . P^{(1-r)}$$

wobei k und r Parameter zum Aufbau der Durchflussbeschränkung (3) sind.

5. Verfahren zur Bewertung des Wobbeindexes nach einem der vorhergehenden Ansprüche, **dadurch gekennzeichnet, dass** das Verfahren zur Messung eine Messung der absoluten Messtemperatur (T1) umfasst, und **dadurch**, dass das Verfahren der Eichung eine Messung der absoluten Referenztemperatur (T0) umfasst.

6. Verfahren zur Bewertung des Wobbeindexes nach einem der vorhergehenden Ansprüche, **dadurch gekennzeichnet, dass** das Referenzgas (G0) frei unter einer Gruppe von Gasen gewählt wird, umfassend Nichtbrennstoff-Gase und -Gasmischungen wie z.B. Luft oder Stickstoff.

7. Verfahren zur Bewertung des Wobbeindexes nach einem der vorhergehenden Ansprüche, **dadurch gekennzeichnet, dass** es ein vorbereitendes Verfahren der Korrelation umfasst, im Laufe dessen das empirische affine Gesetz (W = A X Y + B) für mindestens zwei Gase der Gruppe der Gase festgesetzt wird.

8. Verfahren zur Bewertung des Wobbeindexes nach einem der vorhergehenden Ansprüche, **dadurch gekennzeichnet, dass** zur Bewertung des Wobbeindexes eines Gases der Gruppe von Naturgasen das empirische affine Gesetz (W = A X Y + B) durch eine Ordinate mit dem Ursprung (B) gleich -18,40 definiert wird

9. Verfahren zur Bewertung des Wobbeindexes nach Anspruch 8, **dadurch gekennzeichnet, dass** die Druckluft als Referenzgas (G0) verwendet wird, und **dadurch**, dass das empirische affine Gesetz (W = A X Y + B) durch eine Neigung (A) gleich 19,40 definiert wird.

10. Verfahren zur Bewertung des Wobbeindexes nach Anspruch 8, **dadurch gekennzeichnet, dass** der Stickstoff als Referenzgas (G0) verwendet wird, und **dadurch**, dass das empirische affine Gesetz (W = A X Y + B) durch eine Neigung (A) gleich 19,72 definiert wird.

11. Verfahren zur Bewertung des Wobbeindexes nach Anspruch 8, **dadurch gekennzeichnet, dass** das Methan als Referenzgas (G0) verwendet wird, und **dadurch**, dass das empirische affine Gesetz (W = A X Y + B) durch eine Neigung (A) gleich 33,28 definiert wird.

12. Vorrichtung, angepasst für die Bewertung des Wobbeindexes eines Brennstoffgases (G1), das einer Gruppe von Gasen angehört, definiert durch Bestandteile einer bestimmten chemischen Art, die in variable relative Proportionen eingreifen, wobei das Verfahren eine Leitung (1) umfasst, die einen Eingang (11) und einen Ausgang (12) umfasst, Mittel zum Einlass (21, 22, 81, 82), um selektiv bis zum Eingang (11) der Leitung zu führen, einen Fluss von Brennstoffgas (G1) unter Druck oder einen Fluss von Referenzgas (G0) unter Druck, eine Durchflussbeschränkung (3), wie z.B. eine Öffnung oder eine Mikrodüse, aufweisend einen Eingang (31), der mit dem Ausgang (12) der Leitung verbunden ist, einen Ausgang (32) sowie einen Sensor (5) des absoluten Drucks und einen Sensor (6) der absoluten Temperatur, eingebaut auf der Leitung (1), **dadurch gekennzeichnet, dass** sie einen Massendurchflussmesser (4) umfasst, der am Ausgang (32) der Durchflussbeschränkung (3) durchgeführt ist und ein Ausgangssignal (Qa) bereitstellt, das den Massendurchfluss (4) des Gases, das diese Durchflussbeschränkung in Strömung mit Schallgeschwindigkeit durchfließt, darstellt, ebenso wie eine logische Einheit (7), um die Ausgangssignale (Qm1, Qm0, P1, 0, T1, T0) des Massendurchflusses des Brennstoffgases (G1) und des Massendurchflusses des Referenzgases (G0), des absoluten Messdrucks, des absoluten Referenzdrucks, der absoluten Messtemperatur bzw. der absoluten Referenztemperatur des Brennstoffgases und des Referenzgases zu nutzen.

13. Vorrichtung nach Anspruch 12, **dadurch gekennzeichnet, dass** die Mittel zum Einlass (21, 22, 81, 82) einen ersten Haupteingang (81) für das Brennstoffgas (G1), einen zweiten Haupteingang (82) für das Referenzgas (G0) und erste und zweite Elektroventile (21, 22) umfassen, die jeweils zwischen den Eingang (11) der Leitung (1) und dem ersten und dem zweiten Haupteingang (81, 82) angebracht sind.

14. Vorrichtung nach Anspruch 12 oder 13, **dadurch gekennzeichnet, dass** die logische Einheit (7) mindestens drei Empfangseingänge (71, 72, 73) umfasst, die jeweils mit dem Durchflussmesser (4), dem Drucksensor (5) und den Temperatursensor (6) verbunden sind, und zwei Steuerausgänge (74, 75), die jeweils mit den Elektroventilen (21, 22) verbunden sind.

## RÉFÉRENCES CITÉES DANS LA DESCRIPTION

**Documents brevets cités dans la description**

- DE 4118781
- US 4384792 A
- US 6119710 A